# EUROPEAN PATENT APPLICATION

(11) **EP 4 282 324 A1**
(43) Date of publication of application: **29.11.2023**
(21) Application number: 22920977.0
(22) Date of filing: 20.12.2022
(51) Int. Cl.: A61B 5/00, G04G 21/02

(54) **WEARABLE DEVICE**

(30) Priority: 23.03.2022 CN 202210290839
(71) Applicant: Honor Device Co., Ltd., Shenzhen, Guangdong 518040 (CN)
(72) Inventor: BAI, Liang, Shenzhen, Guangdong 518040 (CN); HUANG, Hua, Shenzhen, Guangdong 518040 (CN); SHANG, Ruiying, Shenzhen, Guangdong 518040 (CN)
(74) Representative: Gill Jennings & Every LLP
(86) International application number: PCT/CN2022/140491
(87) International publication number: WO 2023/179125

(57) **Abstract**

Embodiments of this application provide a wearable device. The wearable device includes at least a housing, a circuit board, a magnetic component, and a vital sign sensor. The housing includes a bottom housing. The circuit board is disposed in the housing. The magnetic component is disposed on a side that is of the circuit board and that faces the bottom housing. The magnetic component includes an accommodation hole. The vital sign sensor is disposed in the housing. The vital sign sensor includes a light emission element and a light receive element. The light emission element and the light receive element are disposed on the side that is of the circuit board and that faces the bottom housing. The light emission element is located in the accommodation hole. The light receive element is located on an outer side that is of the magnetic component and that faces away from the light emission element. According to the wearable device in the embodiments of this application, an overall thickness of the wearable device can be reduced, to facilitate a miniaturization design of the wearable device.

## Description

This application claims priority to Chinese Patent Application No. 202210290839.8, filed with the China National Intellectual Property Administration on March 23, 2022 and entitled "WEARABLE DEVICE", which is incorporated by reference herein in its entirety.

### TECHNICAL FIELD

Embodiments of this application relate to the field of terminal technologies, and in particular, to a wearable device.

### BACKGROUND

With the explosive growth of wearable devices such as smartwatches or smart bands, the wearable devices have increasingly more functions. A battery and a circuit board are disposed in a housing of a wearable device. Different electronic devices are integrated on the circuit board, for example, a wireless charging chip, an intelligent algorithm chip, a power management chip (Power Management IC, PMIC), or a vital sign sensor. The vital sign sensor may be configured to detect vital sign information such as a heart rate, a pulse, or blood oxygen of a user. A charging coil is disposed in the wearable device, so that the wearable device has a wireless charging function. A magnet is disposed in the housing of the wearable device. A magnet is also disposed in a charging dock. When the wearable device is placed on the charging dock for charging, the magnet in the housing and the magnet in the charging dock may attract each other, so that a position of the wearable device can be kept stable, and the wearable device is not easily shaken relative to the charging dock. However, in the wearable device in the related technology, a magnet design is improper. Consequently, an overall thickness of the wearable device is large, and a miniaturization design of the wearable device is affected.

### SUMMARY

Embodiments of this application provide a wearable device, so that an overall thickness of the wearable device can be reduced, to facilitate a miniaturization design of the wearable device.

An embodiment of this application provides a wearable device, including at least a housing, a circuit board, a magnetic component, and a vital sign sensor.

The housing includes a bottom housing. The circuit board is disposed in the housing. The magnetic component is disposed on a side that is of the circuit board and that faces the bottom housing. The magnetic component includes an accommodation hole. The vital sign sensor is disposed in the housing. The vital sign sensor includes a light emission element and a light receive element. The light emission element and the light receive element are disposed on the side that is of the circuit board and that faces the bottom housing. The light emission element is located in the accommodation hole. The light receive element is located on an outer side that is of the magnetic component and that faces away from the light emission element.

According to the wearable device in this embodiment of this application, the magnetic component is disposed between the circuit board and the bottom housing, so that a mounting space between the circuit board and the bottom housing can be fully used. The magnetic component is close to the bottom housing. Therefore, the space between the circuit board and the bottom housing can be fully used for the magnetic component, so that the circuit board can be closer to a display module, to help reduce an overall thickness of the wearable device. In addition, when the wearable device is connected to a charging dock, and it is ensured that a magnetic attraction force generated between the wearable device and the charging dock is unchanged, because a distance between the magnetic component and the charging dock is small, a thickness of the magnetic component may be relatively small, to help reduce the overall thickness of the wearable device, and also help reduce an overall weight of the wearable device.

In a possible implementation, the magnetic component is of an integrated structure, so that overall mechanical strength of the magnetic component is high.

In a possible implementation, the magnetic component includes at least two magnetic bodies and a first connection member. Two adjacent magnetic bodies are connected by using the first connection member. The two magnetic bodies may be separately processed and manufactured, to help reduce processing difficulty of the magnetic bodies, and reduce processing and manufacture difficulty of the magnetic component.

In a possible implementation, the first connection member is a bonding member. The magnetic bodies are bonded, so that a connection structure such as a bracket, a screw, a buckle, or a rivet does not need to be additionally disposed between two adjacent magnetic bodies. Therefore, this helps reduce complexity of the connection structure between the two adjacent magnetic bodies, reduce assembly difficulty, and save a mounting space. In addition, a corresponding connection structure such as a hole does not need to be disposed on two adjacent magnetic bodies, to help reduce processing difficulty of the magnetic bodies, and ensure good structural integrity of the magnetic bodies.

In a possible implementation, the magnetic bodies and the first connection member form the magnetic component by using an injection molding process. The magnetic bodies and the first connection member may form an integral structure, to help improve connection stability and reliability of the magnetic bodies and the first connection member, so that the magnetic bodies and the first connection member are not easily separated.

In a possible implementation, the magnetic component is of a light-blocking structure. The magnetic component in this embodiment of this application may block light emitted in a radial direction of the accommodation hole from being directly incident to the light receive element, to avoid an optical crosstalk case in which light emitted by the light emission element is directly incident to the light receive element, and reduce a possibility that a detection result is distorted due to the optical crosstalk case.

In a possible implementation, the wearable device further includes a second connection member. The second connection member connects the magnetic component and the circuit board. When the magnetic component and the circuit board are assembled, it is convenient to limit the magnetic component by using the second connection member, to ensure an accurate mounting position of the magnetic component.

In a possible implementation, the wearable device further includes a second connection member. The second connection member connects the magnetic component and the bottom housing. When the magnetic component and the bottom housing are assembled, it is convenient to limit the magnetic component by using the second connection member, to ensure an accurate mounting position of the magnetic component.

In a possible implementation, the second connection member is of a light-blocking structure, to avoid a case in which light emitted by the light emission element passes through a gap between the magnetic component and the circuit board or a gap between the magnetic component and the bottom housing, further reduce a possibility that an optical crosstalk case occurs because the light emitted by the light emission element is directly incident to the light receive element, and reduce a possibility that vital sign information obtained through analysis and processing is distorted due to the optical crosstalk case.

In a possible implementation, the second connection member is a bonding member. The magnetic component and the circuit board are bonded or the magnetic component and the bottom housing are bonded, so that a connection structure such as a screw, a buckle, or a rivet does not need to be additionally disposed between the magnetic component and the circuit board or between the magnetic component and the bottom housing. Therefore, this helps reduce complexity of the connection structure between the magnetic component and the circuit board or between the magnetic component and the bottom housing, and reduce assembly difficulty. In addition, a corresponding connection structure such as a hole does not need to be disposed on the magnetic component and the circuit board or on the magnetic component and the bottom housing, to help reduce processing difficulty of the magnetic component and the circuit board or the magnetic component and the bottom housing, and ensure good structural integrity of the magnetic component and the circuit board or the magnetic component and the bottom housing.

In a possible implementation, an orthographic projection of the accommodation hole on the circuit board has a same shape as an orthographic projection of the light emission element on the circuit board. Therefore, when the accommodation hole can avoid the light emission element, a size of the accommodation hole may be designed to be small. Therefore, an overall volume of the magnetic component is large, to help reduce a thickness of the magnetic component when an attraction force of the magnetic component is unchanged.

In a possible implementation, an orthographic projection area of the accommodation hole on the circuit board is greater than an orthographic projection area of the light emission element on the circuit board. Therefore, when the magnetic component and the circuit board are assembled, because a size of the accommodation hole is large, the magnetic component does not easily scratch or collide with the light emission element, to reduce a possibility that the light emission element is damaged. In addition, more light emission elements may be disposed in the accommodation hole of the magnetic component.

In a possible implementation, an outer contour of the magnetic component is circular. An outer peripheral surface of the magnetic component has smooth transition, and there is no protrusion region. Therefore, when the magnetic component and the circuit board are assembled, position interference does not easily occur between the outer peripheral surface of the magnetic component and the light receive element. In addition, when the outer peripheral surface of the magnetic component is in contact with the light receive element, the outer peripheral surface of the magnetic component does not easily scratch or scrape the light receive element, to reduce a possibility that the light receive element cannot be normally used because the light receive element is scratched or damaged.

In a possible implementation, the magnetic component is annular.

In a possible implementation, a thickness of the magnetic component has a value range from 0.5 millimeter to 1.5 millimeters. Therefore, when it is ensured that the magnetic component can generate a magnetic field that meets a requirement, the thickness of the magnetic component is properly designed.

In a possible implementation, the accommodation hole is circular, elliptical, or strip-shaped.

In a possible implementation, a surface that is of the light emission element and that faces the bottom housing is lower than a surface that is of the magnetic component and that faces the bottom housing. When the wearable device is assembled, the light emission element does not easily collide with the bottom housing, and the magnetic component can protect the light emission element at a periphery of the light emission element, to reduce a possibility that the light emission element is damaged. In addition, after the wearable device is assembled, position interference does not easily occur between the light emission element and the bottom housing, to help reduce a possibility that connection reliability between the magnetic component and the bottom housing is affected by position interference caused because the light emission element is in contact with the bottom housing.

In a possible implementation, a surface that is of the light receive element and that faces the bottom housing is lower than a surface that is of the magnetic component and that faces the bottom housing. When the wearable device is assembled, the light receive element does not easily collide with the bottom housing, to reduce a possibility that the light emission element is damaged. In addition, after the wearable device is assembled, position interference does not easily occur between the light receive element and the bottom housing, to help reduce a possibility that connection reliability between the magnetic component and the bottom housing is affected by position interference caused because the light receive element is in contact with the bottom housing.

In a possible implementation, at least two light receive elements are evenly distributed along a circumferential direction of the magnetic component. The two light receive elements can effectively receive light reflected in a plurality of directions, to help obtain more accurate vital sign information.

In a possible implementation, the bottom housing includes a light transmission part. The light emission element and the light receive element separately correspond to the light transmission part.

In a possible implementation, the wearable device further includes a battery. The battery is located on a side that is of the circuit board and that faces away from the bottom housing. The battery may be disposed between the display module and the circuit board. Because the magnetic component is disposed on a side that is of the circuit board and that faces away from the battery, no position interference occurs between the magnetic component and the battery, to reduce a possibility that arrangement of the battery or a size design of the battery is affected because the magnetic component is located on a side that is of the circuit board and that faces the battery, and also reduce a possibility that the battery is short-circuited or dented due to a scratch or an impact that occurs between the magnetic component and the battery when the wearable device encounters an impact such as fall-off, thereby helping improve safety in a use process of the wearable device.

### BRIEF DESCRIPTION OF DRAWINGS

FIG. 1 is a schematic diagram of a use state of a wearable device according to an embodiment of this application;
FIG. 2 is a schematic diagram of a structure of a wearable device according to an embodiment of this application;
FIG. 3 is a schematic diagram of a cross-sectional structure in an A-A direction in FIG. 2;
FIG. 4 is a schematic enlarged view of B in FIG. 3;
FIG. 5 is a schematic diagram of a partial structure of a wearable device according to an embodiment of this application;
FIG. 6 is a schematic diagram of a working state of a wearable device according to an embodiment of this application;
FIG. 7 is a schematic diagram of a partial structure of a wearable device according to another embodiment of this application;
FIG. 8 is a schematic diagram of a structure for simulation of an attraction force between a magnetic component and a magnet of a charging dock according to an embodiment of this application;
FIG. 9 is a schematic diagram of a partial structure of a wearable device according to still another embodiment of this application; and
FIG. 10 is a schematic diagram of a partial structure of a wearable device according to yet another embodiment of this application.

### Reference numerals:

10. wearable device;
20. housing; 21. front housing; 22. bottom housing; 221. light transmission part;
30. circuit board;
40. magnetic component; 40a. accommodation hole; 41. magnetic body; 42. first connection member;
50. vital sign sensor; 51. light emission element; 511. light emitting unit; 52. light receive element;
60. display module;
70. battery;
80. second connection member;
90. main board;
100. skin;
200. charging dock; 210. magnet; and
X. thickness direction.

### DESCRIPTION OF EMBODIMENTS

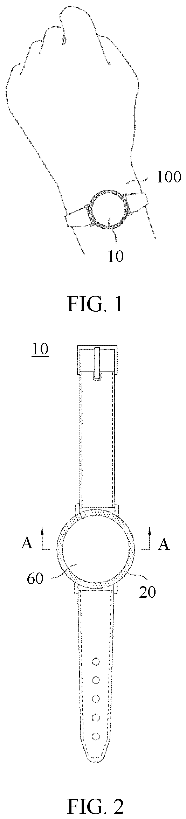

FIG. 1 schematically shows a use state of a wearable device according to an embodiment of this application. Referring to FIG. 1, a wearable device 10 in this embodiment of this application may include a smartwatch or a smart band. A form of the wearable device 10 is not specifically limited in this embodiment of this application. For example, the wearable device 10 in this embodiment of this application may include a smart headset. An implementation of the wearable device 10 provided in this embodiment of this application is described below.

In some possible implementations, the wearable device 10 in this embodiment of this application may include a wrist strap. For example, the wearable device 10 may be worn on a wrist of a user by using the wrist strap. The user may obtain information such as a current time, a date, a position, or a body state by using the wearable device 10, or may make a call, play music, or view information by using the wearable device 10.

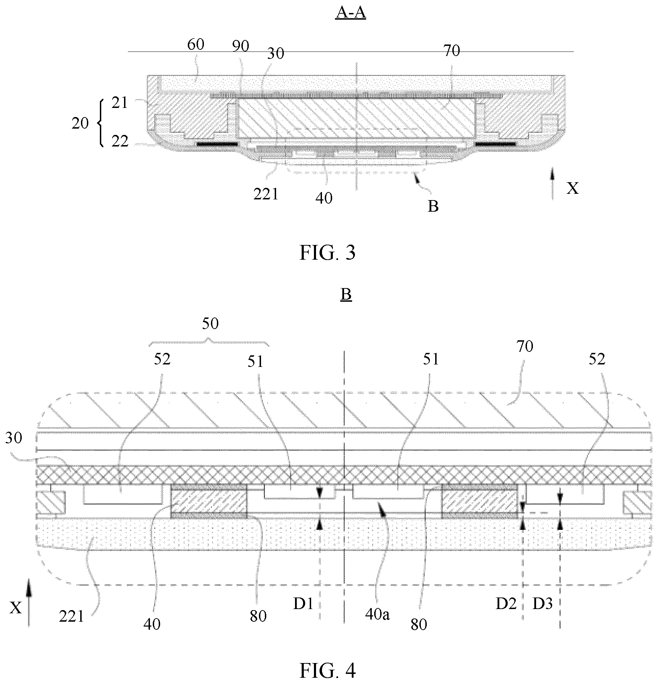

FIG. 2 schematically shows a structure of a wearable device 10 according to an embodiment of this application. Referring to FIG. 2 and FIG. 3, the wearable device 10 in this embodiment of this application may include a housing 20, a circuit board 30, a magnetic component 40, a vital sign sensor 50, a display module 60, a battery 70, and a main board 90.

The housing 20 includes a front housing 21 and a bottom housing 22 that are connected to each other. When the user wears the wearable device 10, the bottom housing 22 of the housing 20 may face skin 100 of the user (as shown in FIG. 1), or may be in contact with skin 100 of the user. The circuit board 30, the magnetic component 40, and the vital sign sensor 50 are disposed in the housing 20. The housing 20 can protect the circuit board 30, the magnetic component 40, and the vital sign sensor 50.

The display module 60 may include a display screen, a touch electrode, and a transparent cover body. The display screen includes a display region used to display image information. The display module 60 is connected to the housing 20. Specifically, the display module 60 may be disposed on the front housing 21. The display region of the display screen is exposed to present image information to the user. The display screen in this embodiment of this application may be but is not limited to a liquid crystal display.

In some possible implementations, the display module 60 may be circular or rectangular. A specific shape of the display module 60 is not limited in this embodiment of this application.

The circuit board 30 is located on an inner side of the display module 60. The circuit board 30 may be located between the display module 60 and the bottom housing 22, so that the user does not easily observe the circuit board 30 from outside of the wearable device 10. The circuit board 30 includes an electronic device (not shown in the figure). The circuit board 30 may be a printed circuit board (Printed Circuit Board, PCB). The electronic device may include but is not limited to a wireless charging chip, an intelligent algorithm chip, or a power management chip (Power Management IC, PMIC). There is a mounting space between the circuit board 30 and the bottom housing 22. In some possible implementations, the circuit board 30 may be bonded to the housing 20. In some examples, a surface that is of the circuit board 30 and that faces the bottom housing 22 may be bonded to the housing 20.

The main board 90 may be disposed inside the housing 20, so that the user does not easily observe the main board 90 from outside of the wearable device 10. The circuit board 30 is electrically connected to the main board 90, so that data can be exchanged between the circuit board 30 and the main board 90. In some possible implementations, the main board 90 may be disposed on a side that is of the display module 60 and that faces the circuit board 30. However, it can be understood that a specific position for disposing the main board 90 is not limited in this embodiment of this application. An electronic device is disposed on the main board 90. For example, the electronic device may include but is not limited to a central processing unit (Central Processing Unit, CPU).

Referring to FIG. 3 and FIG. 4, the magnetic component 40 is disposed on a side that is of the circuit board 30 and that faces the bottom housing 22. In a thickness direction X of the wearable device 10, the magnetic component 40 is located between the circuit board 30 and the bottom housing 22. The magnetic component 40 is connected to the circuit board 30. The magnetic component 40 includes an accommodation hole 40a. For example, in the thickness direction X of the wearable device 10, the accommodation hole 40a may be a through hole that penetrates the magnetic component 40.

It should be noted that the thickness direction X of the wearable device 10 is a direction in which the bottom housing 22, the circuit board 30, and the display module 60 are stacked.

The battery 70 for supplying power and a charging coil are disposed inside the wearable device 10. For example, the battery 70 may be a lithium ion battery. In some examples, the battery 70 may be disposed on a side that is of the main board 90 and that faces away from the display module 60. The wearable device 10 with a wireless charging function may charge the battery 70 without using a wire, to provide use convenience for the user. For example, the wearable device 10 may be connected to an external charging dock, and charge the battery 70 by using the charging coil. When the wearable device 10 is placed on the charging dock for charging, the magnetic component 40 of the wearable device 10 and a magnet in the charging dock may be mutually attracted, and a generated magnetic attraction force can ensure that a position of the wearable device 10 is stable, so that the wearable device 10 is not easily moved relative to the charging dock. When the user needs to use the wearable device 10, an external force may be applied to the wearable device 10, so that the wearable device 10 is separated from the charging dock, and charging is stopped.

In some possible implementations, the vital sign sensor 50 is disposed in the housing 20. The vital sign sensor 50 includes a light emission element 51 and a light receive element 52. The light emission element 51 may emit light to the skin 100 of the user. A part of the light is absorbed by the skin 100 of the user, and another part of the light is reflected by the skin 100 of the user. The reflected part of the light is received by the light receive element 52. In some examples, the vital sign sensor 50 may be a PPG (Photo Plethysmo Graph) sensor.

Both the light emission element 51 and the light receive element 52 of the vital sign sensor 50 are disposed on the side that is of the circuit board 30 and that faces the bottom housing 22. The light emission element 51 is located in the accommodation hole 40a of the magnetic component 40, and the light receive element 52 is located on an outer side that is of the magnetic component 40 and that faces away from the light emission element 51. Therefore, the magnetic component 40 may isolate the light emission element 51 from the light receive element 52.

According to the wearable device 10 in this embodiment of this application, the magnetic component 40 is disposed between the circuit board 30 and the bottom housing 22, so that a mounting space between the circuit board 30 and the bottom housing 22 can be fully used. The magnetic component 40 is close to the bottom housing 22. Therefore, the space between the circuit board 30 and the bottom housing 22 can be fully used for the magnetic component 40, so that the circuit board 30 can be closer to the display module 60, to help reduce an overall thickness of the wearable device 10. In addition, when the wearable device 10 is connected to the charging dock, and it is ensured that a magnetic attraction force generated between the wearable device 10 and the charging dock is unchanged, because a distance between the magnetic component 40 and the charging dock is small, a thickness of the magnetic component 40 may be relatively small, to help reduce the overall thickness of the wearable device 10, and also help reduce an overall weight of the wearable device 10. According to the wearable device 10 provided in this embodiment of this application, the magnetic component 40 is properly disposed in the housing 20, so that the overall thickness of the wearable device 10 can be reduced, to help improve wearing comfort of and satisfaction with the wearable device 10.

According to the wearable device 10 in this embodiment of this application, the magnetic component 40 is disposed on one side of the circuit board 30, and is located between the light emission element 51 and the light receive element 52. The magnetic component 40 may isolate the light emission element 51 from the light receive element 52. Because the magnetic component 40 is disposed on the side that is of the circuit board 30 and that faces the bottom housing 22, a side that is of the circuit board 30 and that faces away from the bottom housing 22 may be used to dispose an electronic device, so that two sides of the circuit board 30 are provided with electronic devices, to help improve utilization of the circuit board 30.

In some possible implementations, the bottom housing 22 of the housing 20 includes a light transmission part 221. The light transmission part 221 has good light transmittance. The magnetic component 40 is disposed on a side that is of the circuit board 30 and that faces the light transmission part 221. The light emission element 51 and the light receive element 52 separately correspond to the light transmission part 221. Light emitted by the light emission element 51 may pass through the light transmission part 221. Light reflected from the skin 100 of the user may pass through the light transmission part 221, and is incident to the light receive element 52. For example, a material of the light transmission part 221 may be plastic or glass. In some examples, the bottom housing 22 may be of an integrally formed structure. A material of the bottom housing 22 may be plastic or glass.

In some possible implementations, the battery 70 in the wearable device 10 may supply power to a component such as the vital sign sensor 50 or the display module 60. The battery 70 is located on the side that is of the circuit board 30 and that faces away from the bottom housing 22. The battery 70 may be disposed between the display module 60 and the circuit board 30. Because the magnetic component 40 is disposed on a side that is of the circuit board 30 and that faces away from the battery 70, no position interference occurs between the magnetic component 40 and the battery 70, to reduce a possibility that arrangement of the battery 70 or a size design of the battery 70 is affected because the magnetic component 40 is located on a side that is of the circuit board 30 and that faces the battery 70, and also reduce a possibility that the battery 70 is short-circuited or dented due to a scratch or an impact that occurs between the magnetic component 40 and the battery 70 when the wearable device 10 encounters an impact such as fall-off, thereby helping improve safety in a use process of the wearable device 10.

In some examples, there may be a gap between the battery 70 and the circuit board 30. Therefore, an electronic device may also be disposed on the side that is of the circuit board 30 and that faces away from the bottom housing 22, to fully use a space between the circuit board 30 and the battery 70, and help improve utilization of the circuit board 30. In addition, the battery 70 does not easily come into contact with the circuit board 30, to reduce a possibility that the battery 70 is short-circuited or dented due to a scratch or an impact that occurs between the circuit board 30 and the battery 70 when the wearable device 10 encounters an impact such as fall-off, thereby improving safety in a use process of the wearable device 10.

In some possible implementations, a surface that is of the light emission element 51 and that faces the bottom housing 22 is lower than a surface that is of the magnetic component 40 and that faces the bottom housing 22. A first distance D1 exists between the surface that is of the light emission element 51 and that faces the bottom housing 22 and an inner surface of the bottom housing 22. A second distance D2 exists between the surface that is of the magnetic component 40 and that faces the bottom housing 22 and the inner surface of the bottom housing 22. The first distance D1 is greater than the second distance D2. When the wearable device 10 is assembled, the light emission element 51 does not easily collide with the bottom housing 22, and the magnetic component 40 can protect the light emission element 51 at a periphery of the light emission element 51, to reduce a possibility that the light emission element 51 is damaged. In addition, after the wearable device 10 is assembled, position interference does not easily occur between the light emission element 51 and the bottom housing 22, to help reduce a possibility that connection reliability between the magnetic component 40 and the bottom housing 22 is affected by position interference caused because the light emission element 51 is in contact with the bottom housing 22.

In some possible implementations, a surface that is of the light receive element 52 and that faces the bottom housing 22 is lower than a surface that is of the magnetic component 40 and that faces the bottom housing 22. A third distance D3 exists between the surface that is of the light receive element 52 and that faces the bottom housing 22 and an inner surface of the bottom housing 22. A second distance D2 exists between the surface that is of the magnetic component 40 and that faces the bottom housing 22 and the inner surface of the bottom housing 22. The third distance D3 is greater than the second distance D2. When the wearable device 10 is assembled, the light receive element 52 does not easily collide with the bottom housing 22, to reduce a possibility that the light emission element 51 is damaged. In addition, after the wearable device 10 is assembled, position interference does not easily occur between the light receive element 52 and the bottom housing 22, to help reduce a possibility that connection reliability between the magnetic component 40 and the bottom housing 22 is affected by position interference caused because the light receive element 52 is in contact with the bottom housing 22.

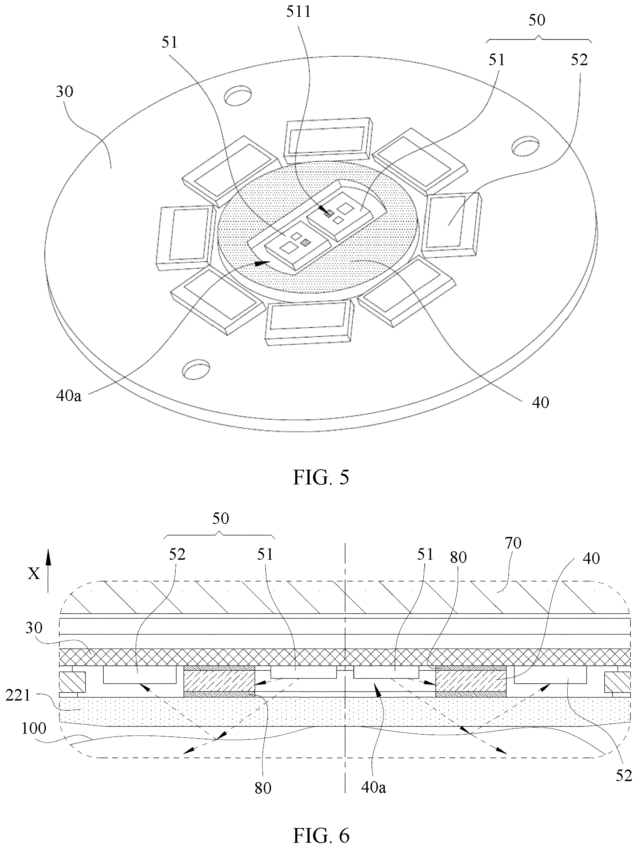

FIG. 5 schematically shows a partial structure of a wearable device 10 according to an embodiment. Referring to FIG. 5, the light emission element 51 includes a light emitting unit 511. The light emitting unit 511 may emit light in an energized state, for example, laser light, visible light (for example, green light or red light), or infrared light. For example, the light emitting unit 511 may include but is not limited to a light emitting diode (light emitting diode, LED). When the vital sign sensor 50 needs to collect vital sign information of the user, the light emitting unit 511 of the light emission element 51 may emit light to the skin 100 of the user. The accommodation hole 40a of the magnetic component 40 may avoid the light emission element 51, to ensure that light emitted by the light emission element 51 is not blocked by the magnetic component 40. A part of the light is absorbed by the skin 100 of the user, and a part of the light is reflected by the skin 100 of the user. The reflected part of the light is received by the light receive element 52 located outside the magnetic component 40.

In some possible implementations, the magnetic component 40 may be of an integrated structure, so that overall mechanical strength of the magnetic component 40 is high. The accommodation hole 40a is formed in a middle region of the magnetic component 40 through processing. The light emission element 51 and the light receive element 52 of the vital sign sensor 50 may be disposed on the circuit board 30 in advance, and then the magnetic component 40 and the circuit board 30 are assembled. In an assembly process, the accommodation hole 40a of the magnetic component 40 is aligned with the light emission element 51 to avoid the light emission element 51. Then, the magnetic component 40 and the circuit board 30 are connected.

In some possible implementations, FIG. 6 schematically shows a working state of a wearable device 10 according to an embodiment. Referring to FIG. 6, the magnetic component 40 may be of a light-blocking structure. When the vital sign sensor 50 detects vital sign information of the user, the light receive element 52 receives the light reflected from the skin 100 of the user. After information about the reflected light is analyzed and processed, the vital sign information of the user can be accurately obtained. The light emitted from the light emission element 51 may propagate in different directions. If the light emitted by the light emission element 51 in a radial direction of the accommodation hole 40a is directly incident to the light receive element 52, the light received by the light receive element 52 includes both the light reflected from the skin 100 of the user and the light directly emitted from the light emission element 51. As a result, the light directly emitted from the light emission element 51 causes interference. Consequently, the vital sign information obtained by analyzing and processing the light received by the light receive element 52 is distorted. This affects accuracy of the vital sign information, and increases a risk that the user misjudges a health status of the user. The radial direction of the accommodation hole 40a is perpendicular to the thickness direction X of the wearable device 10. The magnetic component 40 in this embodiment of this application may block the light emitted in the radial direction of the accommodation hole 40a from being directly incident to the light receive element 52, to avoid an optical crosstalk case in which the light emitted by the light emission element 51 is directly incident to the light receive element 52, and reduce a possibility that a detection result is distorted due to the optical crosstalk case.

In some examples, the magnetic component 40 may be magnetic. For example, the magnetic component 40 may be a magnet. For example, the magnetic component 40 may be a black permanent magnet, so that the magnetic component 40 can effectively block light or absorb light, and a light reflectivity of the magnetic component 40 can also be decreased.

In some possible implementations, referring to FIG. 6, the wearable device 10 further includes a second connection member 80. When the magnetic component 40 and the circuit board 30 are assembled, it is convenient to limit the magnetic component 40 by using the second connection member 80, to ensure an accurate mounting position of the magnetic component 40. When the magnetic component 40 and the circuit board 30 are assembled, the accommodation hole 40a of the magnetic component 40 is aligned with the light emission element 51 to avoid the light emission element 51, and the magnetic component 40 may be connected to the circuit board 30 by using the second connection member 80. In some examples, the second connection member 80 is connected to the surface that is of the circuit board 30 and that faces the bottom housing 22, that is, the second connection member 80 is located on the side that is of the circuit board 30 and that faces the bottom housing 22, so that the second connection member 80 does not occupy a space on the side that is of the circuit board 30 and that faces away from the bottom housing 22.

For example, the second connection member 80 may be a bonding member. For example, the second connection member 80 may be an adhesive or double-sided tape. The magnetic component 40 and the circuit board 30 are bonded, so that a connection structure such as a screw, a buckle, or a rivet does not need to be additionally disposed between the magnetic component 40 and the circuit board 30. Therefore, this helps reduce complexity of the connection structure between the magnetic component 40 and the circuit board 30, and reduce assembly difficulty. In addition, a corresponding connection structure such as a hole does not need to be disposed on the magnetic component 40 and the circuit board 30, to help reduce processing difficulty of the magnetic component 40 and the circuit board 30, and ensure good structural integrity of the magnetic component 40 and the circuit board 30.

In some possible implementations, the second connection member 80 connects the magnetic component 40 and the bottom housing 22. When the magnetic component 40 and the bottom housing 22 are assembled, it is convenient to limit the magnetic component 40 by using the second connection member 80, to ensure an accurate mounting position of the magnetic component 40. The magnetic component 40 and the bottom housing 22 are connected by using the second connection member 80. This can help improve position stability of the magnetic component 40 and the bottom housing 22, and reduce a possibility that the magnetic component 40 relatively moves in the housing 20 when the wearable device 10 encounters fall-off or an impact.

In some examples, the second connection member 80 may be located between the surface that is of the magnetic component 40 and that faces the bottom housing 22 and the inner surface of the bottom housing 22. An outer surface of the second connection member 80 may be aligned with an outer surface of the magnetic component 40. An inner surface of the second connection member 80 may be aligned with an inner surface of the magnetic component 40. The inner surface of the magnetic component 40 is a surface facing the accommodation hole 40a.

In some examples, the second connection member 80 may be a bonding member. For example, the second connection member 80 may be an adhesive or double-sided tape. The magnetic component 40 and the bottom housing 22 are bonded, so that a connection structure such as a screw, a buckle, or a rivet does not need to be additionally disposed between the magnetic component 40 and the bottom housing 22. Therefore, this helps reduce complexity of the connection structure between the magnetic component 40 and the bottom housing 22, and reduce assembly difficulty. In addition, a corresponding connection structure such as a hole does not need to be disposed on the magnetic component 40 and the bottom housing 22, to help reduce processing difficulty of the magnetic component 40 and the bottom housing 22, and ensure good structural integrity of the magnetic component 40 and the bottom housing 22.

In some possible implementations, the second connection member 80 may block a gap between the magnetic component 40 and the circuit board 30 and a gap between the magnetic component 40 and the bottom housing 22. Therefore, the circuit board 30, the second connection member 80, the magnetic component 40, and the bottom housing 22 may form two independent spaces, so that the light emission element 51 and the light receive element 52 are respectively located in the corresponding independent spaces.

In some examples, the second connection member 80 is of a light-blocking structure, to avoid a case in which the light emitted by the light emission element 51 passes through the gap between the magnetic component 40 and the circuit board 30 or the gap between the magnetic component 40 and the bottom housing 22, further reduce a possibility that an optical crosstalk case occurs because the light emitted by the light emission element 51 is directly incident to the light receive element 52, and reduce a possibility that vital sign information obtained through analysis and processing is distorted due to the optical crosstalk case.

For example, the second connection member 80 may be a black structural member, so that the second connection member 80 can effectively block light or absorb light, and a light reflectivity of the second connection member 80 can also be reduced.

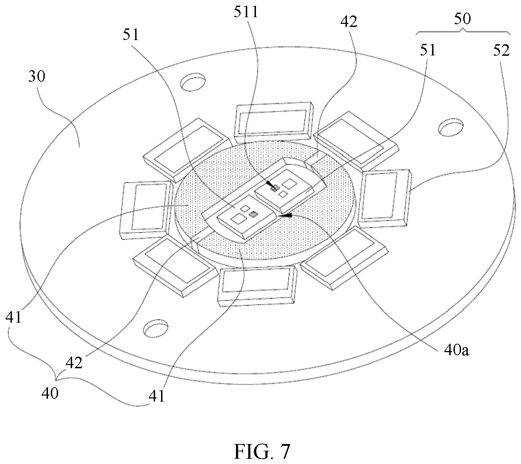

In some possible implementations, FIG. 7 schematically shows a partial structure of a wearable device 10 according to an embodiment. Referring to FIG. 7, the magnetic component 40 includes two magnetic bodies 41 and a first connection member 42. The two magnetic bodies 41 are connected by using the first connection member 42. The two magnetic bodies 41 may be spaced around the light emission element 51. The two magnetic bodies 41 may be separately processed and manufactured, to help reduce processing difficulty of the magnetic bodies 41, and reduce processing and manufacture difficulty of the magnetic component 40. For example, the two magnetic bodies 41 may be symmetric with respect to a central axis of the accommodation hole 40a.

In some examples, a quantity of magnetic bodies 41 is not limited to two, and there may be at least three magnetic bodies. Two adjacent magnetic bodies 41 are connected by using the first connection member 42. For example, at least three magnetic bodies 41 may be evenly distributed around the light emission element 51. In this embodiment of this application, a quantity of magnetic bodies 41 is not specifically limited.

In some examples, the first connection member 42 may be a bonding member. For example, the first connection member 42 may be an adhesive or double-sided tape. The magnetic bodies 41 are bonded, so that a connection structure such as a bracket, a screw, a buckle, or a rivet does not need to be additionally disposed between two adjacent magnetic bodies 41. Therefore, this helps reduce complexity of the connection structure between the two adj acent magnetic bodies 41, reduce assembly difficulty, and save a mounting space. In addition, a corresponding connection structure such as a hole does not need to be disposed on two adjacent magnetic bodies 41, to help reduce processing difficulty of the magnetic bodies 41, and ensure good structural integrity of the magnetic bodies 41.

In some examples, the magnetic bodies 41 and the first connection member 42 may be connected through processing and manufacture by using an injection molding process, to form the magnetic component 40. The magnetic bodies 41 and the first connection member 42 may form an integral structure, to help improve connection stability and reliability of the magnetic bodies 41 and the first connection member 42, so that the magnetic bodies 41 and the first connection member 42 are not easily separated. For example, the first connection member 42 may be wrapped on an outer surface of the magnetic body 41, so that the magnetic bodies 41 can be limited by the first connection member 42. For example, a material of the first connection member 42 may be plastic or rubber.

In some examples, the magnetic bodies 41 may be of a light-blocking structure, so that the magnetic bodies 41 can block light emitted in the radial direction of the accommodation hole 40a from being directly incident to the light receive element 52, to reduce a possibility that an optical crosstalk case occurs because the light emitted by the light emission element 51 is directly incident to the light receive element 52, and reduce a possibility that vital sign information obtained through analysis and processing is distorted due to the optical crosstalk case.

For example, the magnetic bodies 41 may be magnets. For example, the magnetic bodies 41 may be black permanent magnets, so that the magnetic bodies 41 can effectively block light or absorb light, and light reflectivities of the magnetic bodies 41 can also be reduced.

In some examples, the first connection member 42 may be of a light-blocking structure, to avoid a case in which the light emitted by the light emission element 51 passes through a gap between two adjacent magnetic bodies 41, further reduce a possibility that an optical crosstalk case occurs because the light emitted by the light emission element 51 is directly incident to the light receive element 52, and reduce a possibility that vital sign information obtained through analysis and processing is distorted due to the optical crosstalk case.

For example, the first connection member 42 may be a black structural member, so that the first connection member 42 can effectively block light or absorb light, and a light reflectivity of the first connection member 42 can also be reduced.

In some possible implementations, still referring to FIG. 7, in the thickness direction X of the wearable device 10, an orthographic projection area of the accommodation hole 40a of the magnetic component 40 on the circuit board 30 is greater than an orthographic projection area of the light emission element 51 on the circuit board 30. Therefore, when the magnetic component 40 and the circuit board 30 are assembled, because a size of the accommodation hole 40a is large, the magnetic component 40 does not easily scratch or collide with the light emission element 51, to reduce a possibility that the light emission element 51 is damaged. In addition, more light emission elements 51 can be disposed in the accommodation hole 40a of the magnetic component 40.

In some examples, two light emission elements 51 may be disposed in the accommodation hole 40a of the magnetic component 40. However, it can be understood that at least three light emission elements 51 may be disposed in the accommodation hole 40a of the magnetic component 40. This is not specifically limited in this embodiment of this application.

In some examples, an orthographic projection of the accommodation hole 40a on the circuit board 30 may be strip-shaped, and at least two light emission elements 51 may be disposed side by side along a length direction of the accommodation hole 40a. Alternatively, an orthographic projection of the accommodation hole 40a on the circuit board 30 may be circular or elliptical, and at least two light emission elements 51 are arranged in an array in the accommodation hole 40a.

In some possible implementations, an outer contour of the magnetic component 40 is circular. An outer peripheral surface of the magnetic component 40 may be a cylindrical surface. The outer peripheral surface of the magnetic component 40 has smooth transition, and there is no protrusion region. Therefore, when the magnetic component 40 and the circuit board 30 are assembled, position interference does not easily occur between the outer peripheral surface of the magnetic component 40 and the light receive element 52. In addition, when the outer peripheral surface of the magnetic component 40 is in contact with the light receive element 52, the outer peripheral surface of the magnetic component 40 does not easily scratch or scrape the light receive element 52, to reduce a possibility that the light receive element 52 cannot be normally used because the light receive element 52 is scratched or damaged. In some examples, there is a gap between the light receive element 52 and the outer peripheral surface of the magnetic component 40 in the radial direction of the accommodation hole 40a.

In some possible implementations, the magnetic component 40 may be annular. The magnetic component 40 has one accommodation hole 40a. The light emission element 51 disposed on the circuit board 30 is located in the accommodation hole 40a of the magnetic component 40. In some examples, the magnetic component 40 is circular, so that both an outer contour and an inner contour of the magnetic component 40 are circular. A shape of a cross section of the accommodation hole 40a is circular. Alternatively, an outer contour of the magnetic component 40 is circular, and an inner contour thereof is polygonal. For example, the inner contour may be a rectangle whose corners are right angles or a rectangle whose corners are rounded corners. A shape of a cross section of the accommodation hole 40a may be polygonal. For example, the shape of the cross section of the accommodation hole 40a may be a rectangle whose corners are right angles or a rectangle whose corners are rounded corners. Alternatively, an outer contour of the magnetic component 40 is circular, and an inner contour thereof is elliptical. A shape of a cross section of the accommodation hole 40a may be elliptical. In this embodiment of this application, an outer contour shape and an inner contour shape of the magnetic component 40 are not specifically limited.

In some possible implementations, in the thickness direction X of the wearable device 10, when the thickness of the magnetic component 40 is unchanged, a smaller distance between the magnetic component 40 and the magnetic body in the charging dock indicates a larger magnetic attraction force generated between the magnetic component 40 and the magnetic body in the charging dock. In the thickness direction X of the wearable device 10, a larger thickness of the magnetic component 40 indicates larger magnetic field strength of the magnetic component 40. When the distance between the magnetic component 40 and the magnet in the charging dock is fixed and unchanged, a larger thickness of the magnetic component 40 indicates a larger magnetic attraction force generated between the magnetic component 40 and the magnet in the charging dock.

In a related technology, the wearable device 10 includes a magnet used to attract the charging dock. The magnet is disposed on a side that is of the circuit board 30 and that faces the battery 70. As a comparative example, a thickness of the magnet is 1 millimeter. When the magnet is placed on the charging dock, a distance between the magnet and the magnet in the charging dock is 2.172 millimeters, and a generated attraction force is 1.7 N.

In this embodiment of this application, the magnetic component 40 is disposed on the side that is of the circuit board 30 and that faces away from the battery 70. The distance between the magnetic component 40 and the magnet in the charging dock may be smaller. For example, compared with the related technology, the distance between the magnetic component 40 and the magnet in the charging dock may be reduced by 1 millimeter, to help increase the magnetic attraction force, and miniaturize the magnetic component 40.

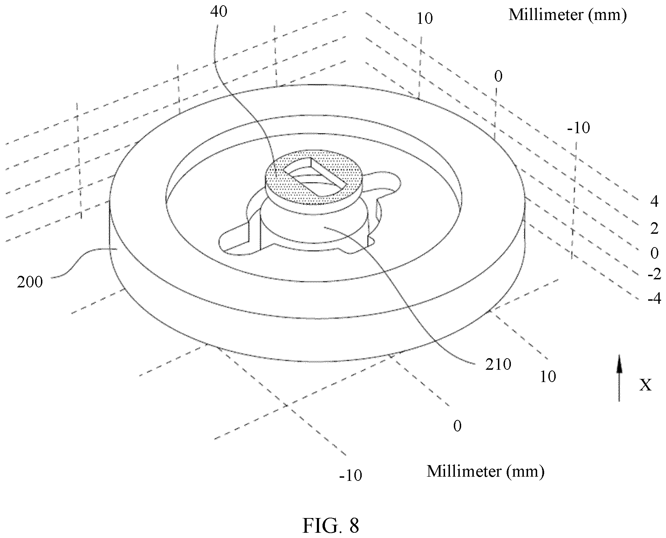

FIG. 8 schematically shows a structure for simulation of an attraction force between a magnetic component 40 and a charging dock 200. Referring to FIG. 8, the following schematically describes two cases to analyze impact on the attraction force.

Case 1: The thickness of the magnetic component 40 is 0.7 millimeter. The thickness of the magnetic component 40 is unchanged (a volume is unchanged). The distance between the magnetic component 40 and the magnet 210 in the charging dock 200 is changed. Simulation data is shown in the following Table 1:

**Table 1:**

| Distance by which a magnetic component moves downward | Distance between the magnetic component and a magnet in a charging dock | X-direction attraction force |
|---|---|---|
| 0 | 2.172 millimeters | 1.3685 N |
| 0.1 millimeter | 2.072 millimeters | 1.4557 N |
| 0.2 millimeter | 1.972 millimeters | 1.6449 N |
| 0.3 millimeter | 1.872 millimeters | 1.7222 N |
| 0.4 millimeter | 1.772 millimeters | 1.8171 N |
| 0.5 millimeter | 1.672 millimeters | 1.9509 N |
| 0.6 millimeter | 1.572 millimeters | 2.1523 N |
| 0.7 millimeter | 1.472 millimeters | 2.2431 N |
| 0.8 millimeter | 1.372 millimeters | 2.2549 N |
| 0.9 millimeter | 1.272 millimeters | 2.4575 N |
| 1 millimeter | 1.172 millimeters | 2.6375 N |

The thickness of the magnetic component in this embodiment of this application is 0.7 millimeter, and is fixed and unchanged. When the distance between the magnetic component 40 and the magnet 210 in the charging dock 200 is reduced by 0.3 millimeter, the attraction force between the magnetic component 40 and the magnet 210 may reach 1.7 N. Therefore, when the magnetic component 40 with a small thickness is used, and the distance between the magnetic component 40 and the magnet 210 in the charging dock 200 is reduced, an attraction force that meets a requirement can be generated, to facilitate a miniaturization design of the magnetic component 40.

Case 2: The distance between the magnetic component 40 and the magnet 210 in the charging dock 200 is unchanged, and an overall thickness of the magnetic component 40 is changed (a volume is changed). Simulation data is shown in the following Table 2:

**Table 2:**

| Overall thickness of a magnetic component | Distance between the magnetic component and a magnet in a charging dock | X-direction attraction force |
|---|---|---|
| 0.7 millimeter | 2.172 millimeters | 1.3853 N |
| 0.8 millimeter | 2.172 millimeters | 1.6969 N |
| 0.9 millimeter | 2.172 millimeters | 1.9240 N |
| 1.0 millimeter | 2.172 millimeters | 2.0078 N |
| 1.1 millimeters | 2.172 millimeters | 2.1771 N |
| 1.2 millimeters | 2.172 millimeters | 2.2024 N |
| 1.3 millimeters | 2.172 millimeters | 2.4059 N |
| 1.4 millimeters | 2.172 millimeters | 2.7412 N |
| 1.5 millimeters | 2.172 millimeters | 2.7451 N |

The distance between the magnetic component 40 and the magnet 210 in the charging dock 200 in this embodiment of this application is unchanged. When the overall thickness of the magnetic component 40 is 0.8 millimeter, the attraction force between the magnetic component 40 and the magnet 210 may reach 1.7 N. Therefore, when the magnetic component 40 with a small thickness is used, an attraction force that meets a requirement can be generated, to facilitate a miniaturization design of the magnetic component 40.

In some possible implementations, in the thickness direction X of the wearable device 10, an orthographic projection of the accommodation hole 40a of the magnetic component 40 on the circuit board 30 has a same shape as an orthographic projection of the light emission element 51 on the circuit board 30. Therefore, when the accommodation hole 40a can avoid the light emission element 51, a size of the accommodation hole 40a may be designed to be small. Therefore, an overall volume of the magnetic component 40 is large, to help reduce a thickness of the magnetic component 40 when an attraction force of the magnetic component 40 is unchanged.

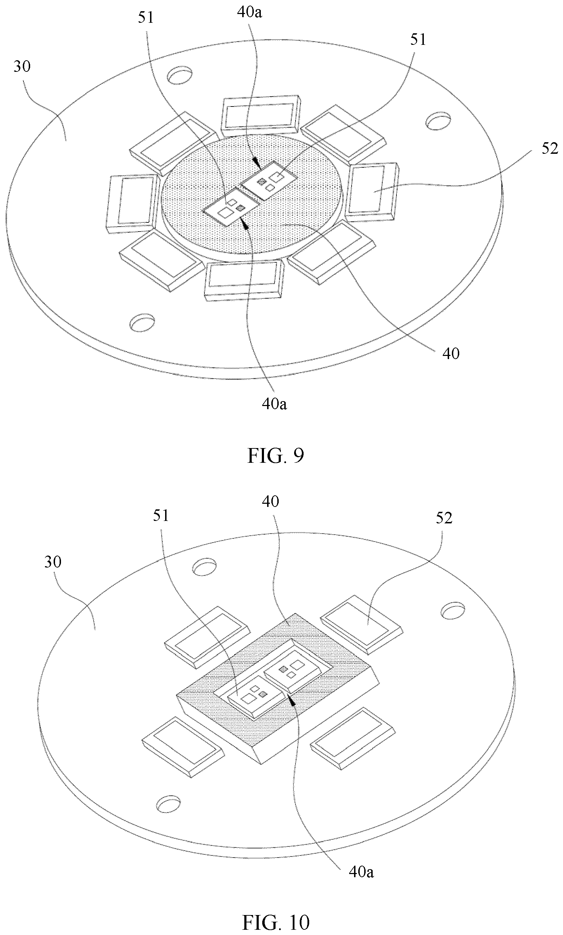

In some examples, a quantity of accommodation holes 40a of the magnetic component 40 may be the same as a quantity of light emission elements 51. For example, FIG. 9 schematically shows a partial structure of a wearable device 10 according to an embodiment. Referring to FIG. 9, there may be two accommodation holes 40a and two light emission elements 51. The two light emission elements 51 are separated from each other by the magnetic component 40. However, it can be understood that the quantity of the accommodation holes 40a and the quantity of the light emission elements 51 are not limited to the foregoing quantities, and there may be one or at least three accommodation holes 40a and there may be one or at least three light emission elements 51.

In some examples, both a shape of the orthographic projection of the accommodation hole 40a on the circuit board 30 and a shape of the orthographic projection of the light emission element 51 on the circuit board 30 may be rectangular or circular.

In some possible implementations, eight light receive elements 52 may be evenly distributed along a circumferential direction of the magnetic component 40. The eight light receive elements 52 can effectively receive light reflected in a plurality of directions, to help obtain more accurate vital sign information. It can be understood that a quantity of light receive elements 52 is not limited to the foregoing quantity, and the quantity of light receive elements 52 may be flexibly set based on an actual product requirement. For example, there may be two to seven light receive elements 52, or there may be at least nine light receive elements 52. This is not specifically limited in this application.

In some possible implementations, FIG. 10 schematically shows a partial structure of a wearable device 10 according to an embodiment. Referring to FIG. 10, the magnetic component 40 may be annular. An outer contour of the magnetic component 40 is rectangular. The accommodation hole 40a of the magnetic component 40 is rectangular. The accommodation hole 40a is provided with two light emission elements 51. Four light receive elements 52 may be distributed along a circumferential direction of the magnetic component 40. It can be understood that a quantity of light emission elements 51 and a quantity of light receive element 52 are not limited to the foregoing quantities, and the quantity of light emission elements 51 and the quantity of light receive elements 52 may be flexibly set based on an actual product requirement. This is not specifically limited in this application.

In the descriptions of the embodiments of this application, it should be noted that unless otherwise specified or limited, terms "mount", "communicate", and "connect" shall be understood in a broad sense, for example, may be a fixed connection, may be an indirect connection by using an intermediate medium, or may be a connection between insides of two elements or an interaction relationship between two elements. A person of ordinary skill in the art can understand specific meanings of the foregoing terms in the embodiments of this application based on a specific situation.

The apparatus or element referred to or implied in embodiments of this application needs to have a specific orientation, be constructed and operated in a specific orientation, and therefore cannot be construed as a limitation on embodiments of this specification. In the descriptions of embodiments of this application, "a plurality of" means two or more, unless otherwise specifically defined.

The terms "first", "second", "third", "fourth", and the like (if existent) in the specification, claims, and accompanying drawings of embodiments of this application are used to distinguish between similar objects, but are not necessarily used to describe a particular order or sequence. It should be understood that the data termed in such a way is interchangeable in proper circumstances, so that embodiments of this application that are described herein can be implemented in orders except the order illustrated or described herein.

Moreover, the terms "include", "contain", and any other variants thereof mean to cover a non-exclusive inclusion. For example, a process, method, system, product, or device that includes a list of steps or units is not necessarily limited to those steps or units that are clearly listed, but may include other steps or units not expressly listed or inherent to such a process, method, product, or device.

Unless otherwise specified, "plurality of" in this specification indicates two or more than two. In this specification, the term "and/or" is merely used to describe an association relationship between associated objects, and indicates that three relationships may exist. For example, "A and/or B" may indicate the following: Only A exists, both A and B exist, and only B exists. In addition, the character "/" in this specification usually indicates an "or" relationship between the associated objects. In a formula, the character "/" indicates that the associated objects are in a "division" relationship.

It may be understood that digital numbers in the embodiments of this application are merely for differentiation for ease of description, and are not intended to limit the scope of the embodiments of this application.

It may be understood that, in the embodiments of this application, sequence numbers of the foregoing processes do not mean execution sequences. The execution sequences of the processes should be determined according to functions and internal logic of the processes, and should not be construed as any limitation on the implementation processes of the embodiments of this application.

## Claims

1. A wearable device, comprising at least:
a housing, comprising a bottom housing;
a circuit board, disposed in the housing;
a magnetic component, disposed on a side that is of the circuit board and that faces the bottom housing, wherein the magnetic component comprises an accommodation hole; and
a vital sign sensor, disposed in the housing, wherein the vital sign sensor comprises a light emission element and a light receive element, the light emission element and the light receive element are disposed on the side that is of the circuit board and that faces the bottom housing, the light emission element is located in the accommodation hole, and the light receive element is located on an outer side that is of the magnetic component and that faces away from the light emission element.

2. The wearable device according to claim 1, wherein the magnetic component comprises at least two magnetic bodies and a first connection member, and two adjacent magnetic bodies are connected by using the first connection member.

3. The wearable device according to claim 2, wherein the first connection member is a bonding member, or the magnetic bodies and the first connection member form the magnetic component by using an injection molding process.

4. The wearable device according to any one of claims 1 to 3, wherein the magnetic component is of a light-blocking structure.

5. The wearable device according to any one of claims 1 to 4, wherein the wearable device further comprises a second connection member, and the second connection member connects the magnetic component and the circuit board; or the wearable device further comprises a second connection member, and the second connection member connects the magnetic component and the bottom housing.

6. The wearable device according to claim 5, wherein the second connection member is of a light-blocking structure.

7. The wearable device according to claim 5 or 6, wherein the second connection member is a bonding member.

8. The wearable device according to any one of claims 1 to 7, wherein an orthographic projection of the accommodation hole on the circuit board has a same shape as an orthographic projection of the light emission element on the circuit board, or an orthographic projection area of the accommodation hole on the circuit board is greater than an orthographic projection area of the light emission element on the circuit board.

9. The wearable device according to any one of claims 1 to 8, wherein an outer contour of the magnetic component is circular.

10. The wearable device according to any one of claims 1 to 9, wherein the magnetic component is annular.

11. The wearable device according to any one of claims 1 to 10, wherein a thickness of the magnetic component has a value range from 0.5 millimeter to 1.5 millimeters.

12. The wearable device according to any one of claims 1 to 11, wherein the accommodation hole is circular, elliptical, or strip-shaped.

13. The wearable device according to any one of claims 1 to 12, wherein a surface that is of the light emission element and that faces the bottom housing is lower than a surface that is of the magnetic component and that faces the bottom housing, or a surface that is of the light receive element and that faces the bottom housing is lower than a surface that is of the magnetic component and that faces the bottom housing.

14. The wearable device according to any one of claims 1 to 13, wherein at least two light receive elements are evenly distributed along a circumferential direction of the magnetic component.

15. The wearable device according to any one of claims 1 to 14, wherein the bottom housing comprises a light transmission part, and the light emission element and the light receive element separately correspond to the light transmission part.

16. The wearable device according to any one of claims 1 to 15, wherein the wearable device further comprises a battery, and the battery is located on a side that is of the circuit board and that faces away from the bottom housing.
